# EUROPEAN PATENT APPLICATION

(11) **EP 4 520 766 A1**
(43) Date of publication of application: **12.03.2025**
(21) Application number: 23799129.4
(22) Date of filing: 14.03.2023
(51) Int. Cl.: C07K 7/08, C07K 16/18, C12N 15/12, A61K 38/10, A61P 3/00, A61P 3/04, A61P 3/06, A61P 3/10, A61P 5/50, A61P 15/00, A61K 47/64

(54) **OVARY-TARGETING POLYPEPTIDE, AND DERIVATIVE AND USE THEREOF**

(30) Priority: 05.05.2022 CN 202210479815
(71) Applicant: Shenzhen Institute of Advanced Technology Chinese Academy of Sciences, Shenzhen, Guangdong 518055 (CN)
(72) Inventor: ZHANG, Jian, Shenzhen, Guangdong 518055 (CN); YANG, Yali, Shenzhen, Guangdong 518055 (CN); ZHAO, Huashan, Shenzhen, Guangdong 518055 (CN); REN, Peigen, Shenzhen, Guangdong 518055 (CN); GE, Lei, Shenzhen, Guangdong 518055 (CN); ZHU, Wen, Shenzhen, Guangdong 518055 (CN); XIAO, Tianxia, Shenzhen, Guangdong 518055 (CN); LI, Mengxia, Shenzhen, Guangdong 518055 (CN)
(74) Representative: Durán-Corretjer, S.L.P.
(86) International application number: PCT/CN2023/081313
(87) International publication number: WO 2023/213141

(57) **Abstract**

Provided are an ovary-targeting polypeptide, a derivative of the polypeptide, and the use of the polypeptide in the preparation of a drug or reagent for treating ovarian related reproductive diseases and preventing and treating the in-vivo or in-vitro aging of oocytes. The diseases comprise menstrual cycle disorder, polycystic ovary syndrome, androgenic phenotype, hair loss, abnormal metabolism, ovarian insufficiency, premature ovarian failure, ovulation failure, abnormal glucose metabolism, and abnormal lipid metabolism, preferably, hyperglycemia and insulin resistance. In addition, the polypeptide is expected to prolong the preservation time and improve the preservation quality of oocytes or fertilized eggs in the field of assisted reproduction.

## Description

### TECHNICAL FIELD

The present invention belongs to the field of medical biology and relates to an ovary-targeting polypeptide, and a derivative and the use thereof.

### BACKGROUND

The ovary is a very important reproductive organ in females, consisting of germ cells (oocytes or ova) and somatic cells (granulosa cells, theca cells, and stromal cells), whose interaction determines the function of the ovary, i.e., secretion of the hormones necessary for follicular development, menstrual/estrous cycle, and maintenance of endocrine health and production of mature ova for fertilization to produce offspring. Abnormalities in ovarian function can lead to a series of ovarian reproductive diseases such as premature ovarian failure (POF), polycystic ovary syndrome (PCOS), ovulation failure, and even ovarian cancer (OC).

Ovarian reproductive diseases can cause hormone disorders in the body, manifesting as a series of abnormalities in glucose and lipid metabolism such as obesity, insulin resistance, fatty liver, and osteoporosis; reproductive abnormalities such as over activation of follicles, reduced ovulation, infertility; and signs of aging such as premature climacteric symptoms. Hyperandrogen in females can lead to a series of androgenic phenotypes such as hirsutism, acne, and alopecia, while hyperestrogen can lead to the occurrence of a series of reproductive tumor diseases such as ovarian cancer, breast cancer, and endometrial cancer, seriously endangering the physical and mental health and life safety of the females.

The window for fertilization after oocyte maturation is very limited. For example, the best time for fertilization and development in mice is within 12 h after ovulation, while in humans it is within 4-12 h after ovulation or oocyte retrieval. Over time, the expelled oocytes enter the aging stage and eventually undergo apoptosis, failing to fertilize. Meanwhile, in assisted reproduction intervention, the inhibition of post-ovulation aging of mammals can improve and increase the fertilization rate and the fertility rate.

At present, the treatment aiming at diseases related to ovarian germ cells and endocrine disorders mainly comprises drug treatment, surgical treatment, and assisted reproduction technology. The biggest problem of drug treatment is the incapability of targeted treatment, as drugs act on the whole body and possibly cause various side effects on other tissues and organs of the body. Surgical treatment and assisted reproduction technology, in addition to having a low success rate, also lack a clear non-invasive real-time observation of ovarian development after treatment. Therefore, it is of great social significance and broad economic market to screen out polypeptides capable of specifically targeting the ovary *in vivo,* and to research their role in ovarian reproductive diseases and their use as drug carriers for targeted treatment of ovarian reproductive diseases.

### SUMMARY

In view of the bottleneck problem of treatment for ovarian reproductive diseases mentioned in the background section, the present disclosure aims to provide the function and use of an ovary-targeting polypeptide and a derivative thereof.

The present disclosure has identified the following sequence polypeptides which can specifically target the ovary and alleviate symptoms caused by various ovary-related reproductive diseases, abnormal metabolism, and aging induced by hormone imbalances.

One aspect of the present disclosure provides an ovary-targeting polypeptide, wherein the polypeptide sequence comprises a sequence represented by formula M1-Za-M2 or Za, wherein:
M1 and M2 are each independently a single or multiple amino acid polypeptide segment or are absent; and
Za is Tyr-Leu-X1-X2-X3-X4-Gly-Ala-X5-X6-Pro-X7-Pro-Asp-X8-Leu-Glu-Pro-Thr, wherein
X1 is Asn or Tyr or Asp or is absent,
X2 is Asn or Gln or His or Pro or Ser or is absent,
X3 is Gly or Trp or is absent,
X4 is Leu or is absent,
X5 is Pro or Ser,
X6 is Ala or Val,
X7 is Tyr or Ser or Ala, and
X8 is Pro or Thr,
further, wherein Za is selected from one of:

| | |
|---|---|
| YLGASVPSPDPLEPT | SEQ ID NO: 1; |
| YLNNGLGAPAPYPDPLEPT | SEQ ID NO: 2; |
| YLYQWLGAPVPYPDPLEPT | SEQ ID NO: 3; |
| YLYQWLGAPVPYPDTLEPT | SEQ ID NO: 4 |
| YLYQWLGAPAPYPDPLEPT | SEQ ID NO: 5 |
| YLDHWLGAPAPYPDPLEPT | SEQ ID NO: 6 |
| YLDPGLGAPAPYPDPLEPT | SEQ ID NO: 7 |
| YLDHGLGAPAPYPDPLEPT | SEQ ID NO: 8 |
| YLDQGLGAPAPAPDPLEPT | SEQ ID NO: 9 |
| and YLDSGLGAPVPYPDPLEPT | SEQ ID NO: 10 |

Another aspect of the present disclosure provides a derivative of an ovary-targeting polypeptide, wherein the derivative is a product obtained by performing a conventional modification on an amino acid side chain group, an amino terminal, and a carboxyl terminal of the polypeptide, or a product obtained by attaching a tag for peptide or protein detection or purification to the polypeptide, or a product obtained by performing isotope labeling modification; and the conventional modification is fluorophore modification, phosphorylation modification, disulfide bond cyclization modification, biotin labeling modification, photosensitizer modification, azide modification, PEG modification, methylation modification, fluorescence quencher modification, protein conjugation modification, small molecule compound modification, amination modification, amidation modification, hydroxylation modification, carboxylation modification, carbonylation modification, alkylation modification, acetylation modification, esterification modification, or glycosylation modification.

Further, the tag is His₆, GST, EGFP, MBP, Nus, HA, IgG, FLAG, c-Myc, or ProfinityeXact.

Further, the modification on the amino terminal and the carboxyl terminal is selected from an N-terminal acetylation modification and a C-terminal amination modification of the polypeptide.

Further, the modification on the side chain is selected from a modification on R group of the amino acid side chain in the polypeptide.

Further, a fluorescent dye used in the fluorophore modification is selected from AMCA, FITC, Rhodamine, Cy3, Cy5, Cy5.5, Cy7, AIE, and ICG, and the modification can be used for fluorescence detection.

Further, the phosphorylation modification is selected from a combination of one or more of p-Ser, p-Thr, and p-Tyr.

Further, the glycosylation modification is selected from a combination of one or more of Ser, Asn, Thr, and Tyr.

Further, the nitration modification is selected from a combination of one or more of Tyr.

Further, the biotin in the biotin labeling is selected from D-biotin, biotin hydrazide, photoreactive biotin, and biotin-dUTP.

Further, the photosensitizer modification can be used for preparing a photosensitive formulation.

Further, the azide modification can be used for a secondary ligation reaction.

Further, the PEG modification can be used for preparing a drug carrier.

Further, an isotope used in the isotope labeling is selected from a combination of one or more of 13C, 14C, 14N, 15N, 2H, 3H, 180, 32P, 32S, 34S, 35S, 36S, 35Cl, 37Cl, 1251, and 131I.

A further aspect of the present disclosure provides a polynucleotide encoding the polypeptide or the derivative thereof described above.

A further aspect of the present disclosure provides a vector comprising the polynucleotide described above.

A further aspect of the present disclosure provides a host cell transfected with the vector described above.

A further aspect of the present disclosure provides use of the polypeptide or the derivative thereof described herein in preparing an ovary-targeting detection reagent, ovary-targeting drug, or ovary-targeting tool.

Further, the tool is selected from a carrier or a formulation, for example, a carrier, a formulation, or a kit for achieving a targeting effect by conjugating the polypeptide or the derivative thereof described herein, and the formulation is a liposome, a nanoparticle, etc.

Another aspect of the present disclosure provides the use of the polypeptide or the derivative thereof described above in preparing a drug or a detection reagent capable of targeting ovaries.

Further, the drug and the detection reagent include detection products prepared using the polypeptide and the derivative taking the polypeptide as a framework described above as an antigen, such as detection reagents, kits, and test strips.

Another aspect of the present disclosure provides an ovary-targeting carrier, and the polypeptide and the derivative taking the polypeptide as a framework described above can be used as a tool for targeted treatment of an ovary-related reproductive disease.

Further, the carrier is selected from a liposome, a nanoparticle, an exosome, a biodegradable high molecular substance, and an antibody-ligand.

Further, the carrier contains a component for treating an ovary-related reproductive disease.

The polypeptide and the derivative taking the polypeptide as a framework can be autonomously synthesized by general chemical laboratory conditions, synthesized by recombinant proteins that contain the polypeptide described above and are expressed by genes, and can also be industrially synthesized by a commercial reagent company. A polypeptide is synthesized by adopting a solid phase method, in which different amino acids on the resin are subjected to a condensation reaction to directionally synthesize an amino acid chain. A polypeptide derivative is obtained by labeling a modification group after the amino acids are linked.

A further aspect of the present disclosure provides an ovary-targeting drug, wherein the drug is a conjugate of the polypeptide and the derivative taking the polypeptide as a framework described above with an active component. The polypeptide and the derivative taking the polypeptide as a framework can treat diseases related to reproduction, metabolism, and aging caused by germ cell abnormalities and hormone disorders by targeting the ovary.

A further aspect of the present disclosure provides an ovary-targeting polypeptide probe, wherein the polypeptide probe is prepared by conjugating the polypeptide or the derivative taking the polypeptide as a framework described above with a fluorescent dye through an organic chemical reaction.

Further, the fluorescent dye is selected from dyes having a fluorophore in the near-infrared region, and preferably, the dyes having a fluorophore in the near-infrared region are selected from AIE, Cy5, Cy7, and ICG.

A further aspect of the present disclosure provides use of the polypeptide in preparing a drug or reagent for treating an ovary-related reproductive disease.

Further, the ovary-related reproductive disease is selected from an ovary-related reproductive disease caused by germ cell abnormality or hormone disorder.

Further, the hormone disorder is an above-normal level of androgen.

Further, the ovary-related reproductive disease caused by the germ cell abnormality refers to an ovary-related reproductive disease caused by oocyte aging and/or zona pellucida sclerosis of oocytes, and preferably, the ovary-related reproductive disease caused by the germ cell abnormality is selected from abnormal oocyte number, decreased oocyte quality, and ovum aging.

Further, the ovary-related reproductive disease caused by the hormone disorder includes menstrual cycle disorder, polycystic ovary syndrome, androgenic phenotype, hair loss, abnormal metabolism, ovarian insufficiency, premature ovarian failure, and ovulation failure.

Further, the abnormal metabolism caused by the hormone disorder is selected from abnormal glucose metabolism, abnormal lipid metabolism, and preferably is hyperglycemia, insulin resistance, obesity, fatty liver, and osteoporosis.

Further, the androgenic phenotype is selected from acne, hirsutism, and alopecia.

Further, the aging and related symptoms caused by the hormone disorder include perimenopausal period, climacteric syndrome, aging, acne, pimple formation, hirsutism, and alopecia.

A further aspect of the present disclosure provides use of the polypeptide in preparing a drug or reagent for preventing and treating oocyte aging *in vivo* or *in vitro.*

A further aspect of the present disclosure provides an assisted reproduction article comprising the polypeptide or the derivative thereof described herein.

Further, the assisted reproduction article is a protective solution for oocytes and/or fertilized ova in the assisted reproduction process.

A further aspect of the present disclosure provides use of the assisted reproduction article described above in preparing a protective solution for preserving oocytes and/or fertilized ova in the assisted reproduction process.

The present disclosure has the following beneficial effects:
(1) The candidate polypeptide and the derivative taking the candidate polypeptide as a framework provided herein can specifically target ovaries *in vivo* and *in vitro.*
(2) The candidate polypeptide and the derivative taking the candidate polypeptide as a framework provided herein can be used as a modified polypeptide to enhance the targeting and intervention as well as treatment effects of a drug or a drug carrier on ovaries.
(3) The candidate polypeptide and the derivative taking the candidate polypeptide as a framework provided herein are easy to synthesize and modify and low in cost, thereby having a wide range of applications.
(4) The candidate polypeptide and the derivative taking the candidate polypeptide as a framework provided herein offer a wide range of strategies for the preparation of diagnostic compounds or drugs for their practical applications.
(5) The candidate polypeptide and the derivative taking the candidate polypeptide as a framework provided herein are suitable for being applied in the targeted treatment of ovary-related reproductive diseases, and are also suitable for being used as a research and observation tool in experimental animals modeled for ovarian diseases.

### DESCRIPTION OF THE DRAWINGS

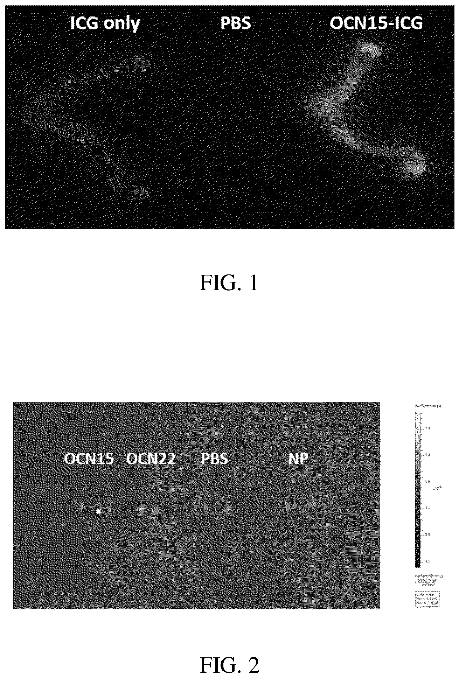

FIG. 1 is a figure showing the ovary-targeting effect of the ICG-linked polypeptide *in vivo.* The figure shows the distribution and results of fluorescent signals from the ovaries and uterus of the three groups of mice: the PBS group, the ICG group, and the ICG-linked polypeptide group.
FIG. 2 is a figure showing the ovary-targeting effect of the AIE-linked polypeptide *in vivo.* The figure shows the distribution and results of fluorescent signals from the ovaries of the four groups of mice: the PBS group, the AIE empty particle group, the AIE-linked polypeptide group, and the AIE-linked additional polypeptide group.

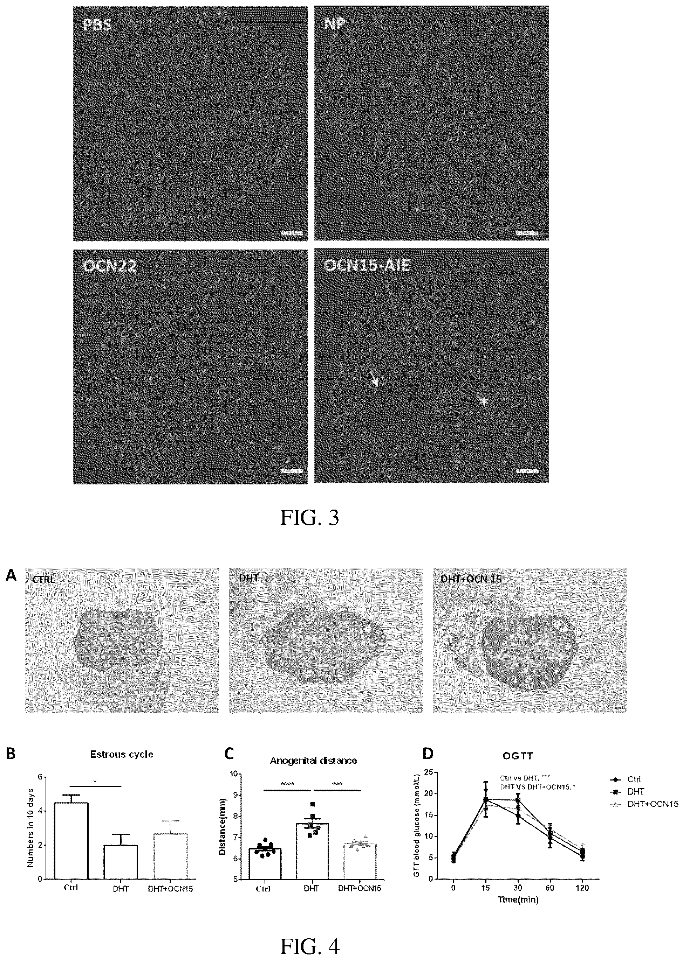

FIG. 3 is a figure showing the ovary-targeting location of the AIE-linked polypeptide *in vivo.* The figure shows the localization of fluorescent signals from the ovaries of the four groups of mice: the PBS group, the AIE empty particle group, the AIE-linked polypeptide group, and the AIE-linked additional polypeptide group. The arrow indicates the membrane cell and the asterisk indicates the interstitial cell.
FIG. 4 shows the alleviation effect of the ovary-targeting polypeptide on follicular dysplasia, estrous cycle disorder, mouse androgenic phenotype, and blood glucose abnormality caused by hyperandrogen in a hyperandrogen-dihydrotestosterone (DHT)-induced mouse model. FIG. 4A is a morphological observation of ovaries of the three groups of mice: the control group, the DHT group, and the DHT + ovary-targeting polypeptide group. FIG. 4B shows the number of estrous days in ten days for the three groups of mice. FIG. 4C shows the anogenital distance (a greater anogenital distance in the male mice) of the mice 60 days after modeling. FIG. 4D shows the changes in blood glucose in mice after oral administration of glucose 60 days after modeling.

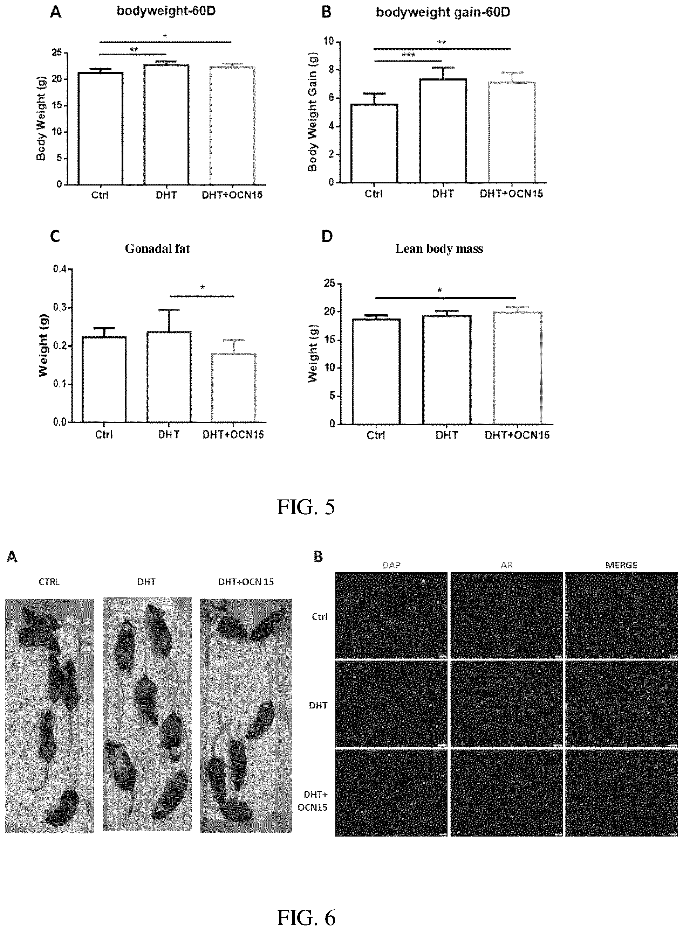

FIG. 5 shows the alleviation effect of the ovary-targeting polypeptide on the changes in body weight caused by hyperandrogen in a hyperandrogen-dihydrotestosterone (DHT)-induced mouse model. FIG. 5A shows the changes in body weight, and FIG. 5B shows the changes in body weight gain. FIG. 5C shows the weight changes in gonadal fat, and FIG. 5D shows the lean body mass changes in addition to fat.
FIG. 6 shows the effect of the ovary-targeting polypeptide on hair loss on the mouse neck and back caused by hyperandrogen in a hyperandrogen-dihydrotestosterone (DHT)-induced mouse model. FIG. 6A is an observation of hair loss on the neck and back of the three groups of mice: the control group, the DHT group, and the DHT + ovary-targeting polypeptide group. FIG. 6B is an immunofluorescence map of the androgen receptor AR expression in the neck and back skin of the three groups of mice.

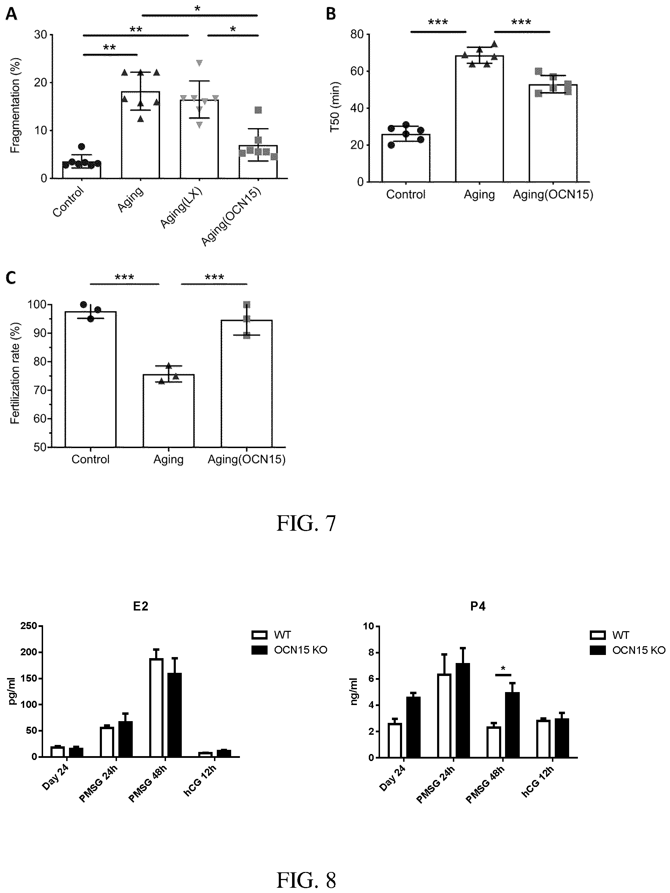

FIG. 7 is the ameliorating effect of the ovary-targeting polypeptide on oocyte fragmentation and degeneration, zona pellucida sclerosis, and *in vitro* fertilization caused by aging in an oocyte post-ovulation aging model. Fig. 7A shows the degeneration and fragmentation rates after 12 h of aging for the Control group, the Aging group, and the ovary-targeting polypeptide group. FIG. 7B shows the zona pellucida sclerosis levels after 12 h of aging for the Control group, the Aging group, and the ovary-targeting polypeptide group. FIG. 7C shows the *in vitro* fertilization rates after 12 h of *in vitro* aging for the Control group, the Aging group, and the ovary-targeting polypeptide group.
FIG. 8 shows the changes in serum estrogen and progesterone levels in the superovulation model in the OCN15 KO mice compared with the WT mice. The left panel of FIG. 8 shows the changes in estrogen level during superovulation. The right panel of FIG. 8 shows the changes in progestogen level during superovulation.

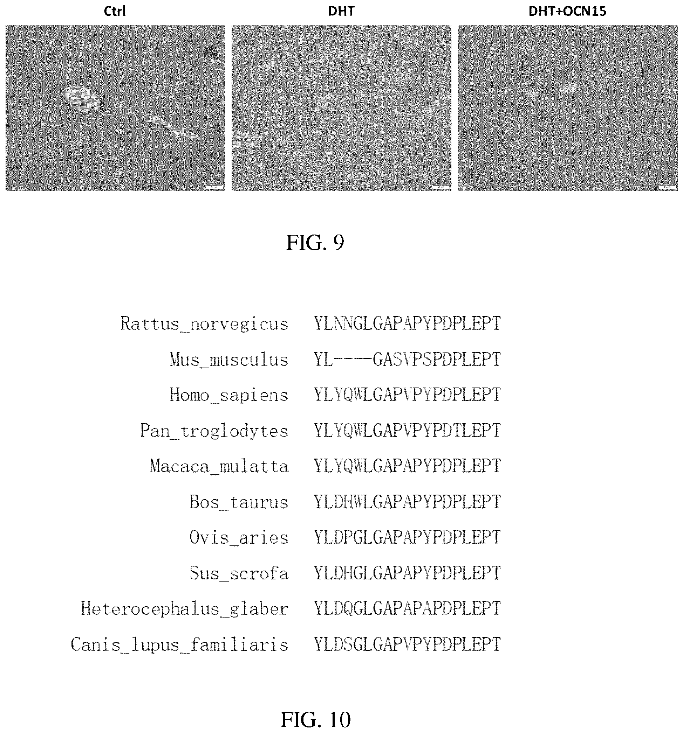

FIG. 9 shows the effect of the ovary-targeting polypeptide on mouse liver lipid droplet accumulation caused by hyperandrogen in a hyperandrogen-dihydrotestosterone (DHT)-induced mouse model.
FIG. 10 is a figure showing the sequence alignment of metabolites from different species.

### DETAILED DESCRIPTION

For a better understanding of the present disclosure, the content of the present disclosure will be further described below with reference to the specific examples, but the protection content of the present disclosure is not limited to the following examples.

Unless otherwise specifically defined, all technical and scientific terms used herein have the same meaning as commonly understood by those of ordinary skill in the art to which the present disclosure belongs. In addition, any method or material similar or equivalent to those described herein can be used in the practice of the present disclosure. For the purposes of the present disclosure, the following terms are defined.

The terms "subject", "individual", and "patient" are used interchangeably herein to refer to a vertebrate, preferably a mammal. The mammal includes, but is not limited to, humans, mice, rats, chimpanzees, macaques, cattle, sheep, boars, moles, and dogs. Also encompassed are tissues, cells, and progeny thereof of biological entities obtained *in vivo* or cultured *in vitro.*

As used herein, the term "administration" includes oral administration, local contact, administration as a suppository, and intravenous, intraperitoneal, intramuscular, intralesional, intrathecal, intranasal, or subcutaneous administration to a subject. The administration is by any route, including parenteral and transmucosal (e.g., transbuccal, sublingual, transpalatal, transgingival, transnasal, transvaginal, transrectal, or transdermal) routes. Parenteral administration includes, for example, intravenous, intramuscular, intraarterial, intradermal, subcutaneous, intraperitoneal, intraventricular, and intracranial administration. Other modes of delivery include, but are not limited to, the use of liposomal formulations, intravenous infusion, transdermal patches, and the like.

The term "treatment" refers to a method for obtaining a beneficial or desired result, including but not limited to a therapeutic benefit. Therapeutic benefit refers to any treatment-related improvement in, or any treatment-related effect on, one or more diseases, conditions, or symptoms under treatment. "Prevention" refers to a method for obtaining a beneficial or desired result, including but not limited to a prophylactic benefit. For the prophylactic benefit, a composition may be administered to a subject at risk of developing a particular disease, condition, or symptom, or to a subject reporting one or more physiological symptoms of a disease, even though the disease, condition, or symptom may not have yet been manifested.

The term "therapeutically effective amount" refers to an amount of a peptide or composition sufficient to achieve a beneficial or desired result. The therapeutically effective amount may vary depending on one or more of the following: a subject and disease condition being treated, the weight and age of the subject, the severity of the disease condition, the mode of administration, and the like, and can be readily determined by those of ordinary skill in the art. The specific amount may vary depending on one or more of the following: the particular agent selected, the type of target cell, the location of the target cell within the subject, the dosage regimen to be followed, whether or not to be administered in combination with other compounds, the time of administration, and the entity delivery system carrying it.

### Example 1: Preparation of Polypeptide Sequences

The polypeptide sequences were synthesized by adopting an artificial synthesis method, and the polypeptide sequences are as follows:

| | |
|---|---|
| YLGASVPSPDPLEPT | SEQ ID NO: 1; |
| YLNNGLGAPAPYPDPLEPT | SEQ ID NO: 2; |
| YLYQWLGAPVPYPDPLEPT | SEQ ID NO: 3; |
| YLYQWLGAPVPYPDTLEPT | SEQ ID NO: 4 |
| YLYQWLGAPAPYPDPLEPT | SEQ ID NO: 5 |
| YLDHWLGAPAPYPDPLEPT | SEQ ID NO: 6 |
| YLDPGLGAPAPYPDPLEPT | SEQ ID NO: 7 |
| YLDHGLGAPAPYPDPLEPT | SEQ ID NO: 8 |
| YLDQGLGAPAPAPDPLEPT | SEQ ID NO: 9 |
| and YLDSGLGAPVPYPDPLEPT | SEQ ID NO: 10 |

The polypeptides described above were synthesized by the conventional solid-phase synthesis or liquid-phase synthesis method. In the solid-phase synthesis of the polypeptides, the reaction proceeded from the C-terminal amino acid towards the N-terminal one, with each amino acid being successively coupled through the steps of resin activation, amino acid coupling, elution protection, detection, and the like. Then precipitation was performed using an excess of ether, followed by centrifugation. After being purified by HPLC, the crude peptides were subjected to mass spectrometry analysis, and quickly freeze-dried in liquid nitrogen for later use.

In the present disclosure, the peptides are all isolated peptides.

SEQ ID NO: 1 is a mouse metabolite, SEQ ID NO: 2 is a rat metabolite, SEQ ID NO: 3 is a human metabolite, SEQ ID NO: 4 is a chimpanzee metabolite, SEQ ID NO: 5 is a macaque metabolite, SEQ ID NO: 6 is a bovine metabolite, SEQ ID NO: 7 is a sheep metabolite, SEQ ID NO: 8 is a boar metabolite, SEQ ID NO: 9 is a mole rat metabolite, and SEQ ID NO: 10 is a dog metabolite (as shown in FIG. 10).

### Example 2: Preparation of Polypeptide Probe

This example provides a polypeptide probe using the polypeptide prepared in Example 1 to bind to ICG/AIE by an organic chemical reaction.

The specific method was commissioned synthesis or self-synthesis. In this example, the probe preparation was completed by linking the polypeptide to ICG or AIE by click chemistry, where the linker was a general DBCO. ICG/AIE with activated functional groups, which include amino NH₂, carboxyl COOH, activated lipid NHS, maleimide MAL, thiol SH, azide N₃, and alkyne ALK, was then conjugated with the polypeptide prepared in Example 1 to obtain the polypeptide ICG/AIE probe.

### Example 3: Detection for Mouse Ovary-Targeting Capacity of Polypeptide ICG Probe

A solution of the polypeptide ICG probe (containing SEQ ID NO: 1) prepared in Example 2, an ICG solution (2 mM), and PBS were injected intravenously into the tail of the adult mice at 100 µL/mouse. After 24 h, the mice were detected under an imager for small animals.

The experimental results are shown in FIG. 1, and it can be seen from the comparison of the results of the 3 groups that the polypeptide ICG probe group (OCN15-ICG, ICG probe containing SEQ ID NO: 1) has a specific signal at the ovarian site, while the other two groups (the ICG solution group and the PBS group) do not show a fluorescent signal at the ovarian site.

The above experimental results show that the polypeptide of the present disclosure has a specific response to the ovary and can realize an ovary-targeting effect *in vivo.*

### Example 4: Detection for Mouse Ovary-Targeting Capacity of Polypeptide AIE Probe

The polypeptide AIE probe (containing SEQ ID NO: 1) prepared in Example 2, a scrambled polypeptide AIE probe, an AIE empty particle, and PBS were injected intravenously into the tail of the adult mice at 100 µL/mouse. After 24 h, the mice were detected under an imager for small animals.

The experimental results are shown in FIG. 2, and it can be seen from the comparison of the results of the 4 groups that the polypeptide AIE probe group (OCN15, AIE probe containing SEQ ID NO: 1) has a specific signal at the ovarian site, while the scrambled polypeptide AIE probe group (OCN22) and the PBS group do not show a fluorescent signal at the ovarian site, and the AIE empty particle group (NP) has a weak signal.

The above experimental results show that the polypeptide of the present disclosure has a specific response to the ovary and can realize an ovary-targeting effect *in vivo.*

As can be seen from the comparison of Examples 3 and 4, the polypeptide of the present disclosure has a specific response to the ovary, and the modification performed on it does not affect its targeting effect, which indicates that the polypeptide of the present disclosure can be used for preparing an ovary-targeting drug, detection reagent, or formulation.

### Example 5: Localization for Targeting of Polypeptide to Mouse Ovary

To further confirm the localization for the targeting of the polypeptide to the ovary, the four groups of mouse ovaries from Example 4 were fixed with 4% PFA, then cryosectioned (10 µm), stained for cell nuclei (DAPI), and observed for fluorescence localization under a laser confocal microscope.

The experimental results are shown in FIG. 3, in which the arrow indicates the membrane cell and the asterisk indicates the interstitial cell.

As can be seen from the comparison of the results of the 4 groups, the polypeptide AIE probe group (OCN15-AIE) mainly targets the interstitial cell and the membrane cell in the mouse ovary.

In conclusion, the mediating polypeptide provided herein can be used for ovarian targeting purposes, in particular for targeting ovarian interstitial cells and membrane cells in the ovary. The modification on the polypeptide does not affect the action of the polypeptide.

The results of Example 5 can prove that OCN15 targets ovarian interstitial cells and membrane cells, and by combining with the results of the following examples, it can be expected to be applied to ovarian diseases related to the two cell abnormalities, such as membrane cell thickening and interstitial cell hyperplasia caused by hyperandrogen, ovulation failure caused by membrane cell thickening and interstitial hyperplasia, and ovarian insufficiency, menstrual cycle disorder, polycystic ovary syndrome, etc. caused by ovulation failure.

### Example 6: Effect of Ovary-Targeting Polypeptide on Hyperandrogen-Induced Reproductive and Metabolic Symptoms in Mice

Wild type female mice at postnatal day 25 (D25) were randomly grouped into a control group (CTRL), a DHT group, and a DHT + OCN15 (OCT15 is the polypeptide set forth in SEQ ID NO: 1) group, with at least 8 mice in each group. The control group was implanted subcutaneously with an empty pump and gavaged with 200 µL of normal saline every day; the DHT group was implanted subcutaneously with a slow-release pump containing 5 mg of dihydrotestosterone (DHT) powder and gavaged with 200 µL of normal saline every day; and the DHT + OCN15 group was implanted subcutaneously with a slow-release pump containing 5 mg of DHT powder and gavaged with 200 µL of normal saline containing the polypeptide (500 µg/Kg) every day. The modeling time was 60 days.

The results are shown in FIG. 4, and it can be seen from the comparison of the 3 groups of mice that hyperandrogen will induce ovarian polycystic changes and decreased estrous cycle in the mice. The estrous days of the normal mice were above 4 days, while the estrous days of the hyperandrogenic mice were around 2 days. However, the concurrent use of OCN15 can alleviate these phenomena to some extent, with estrous days approaching 3 days, and also reduce the polycystic changes (FIGs. 4A and 4B). Hyperandrogen will result in virilization in female mice with increased anogenital distance, which was significantly improved by OCN15 (FIG. 4C), restoring the distance to a level close to that of normal mice. The significant differences in the anogenital distance are shown between the control and DHT groups, as well as between the DHT and DHT + OCN15 groups.

The blood glucose test results are shown in FIG. 4D, where the peak value of blood glucose should have been reached 15 min after oral administration of glucose, and by 30 min, the blood glucose level of the control group had already decreased, while the blood glucose level of the DHT group remained high, showing a difference of 3 stars compared with the control group. After OCN15 treatment, there was a significant decrease in blood glucose level compared with the group with DHT alone, but no difference from the control group, indicating that OCN15 normalizes the blood glucose abnormalities induced by DHT at 30 min.

The results of body weight change are shown in FIG. 5, and FIGs. 5A and 5B show that OCN15 can alleviate the increase in body weight and body weight gain caused by DHT to some extent, mainly reducing the weight of mouse gonadal fat (FIG. 5C). The mice treated with OCN15 in combination with DHT had increased lean body mass, which is predominantly the weight of the skeleton, indicating that OCN15 is able to alleviate osteoporosis caused by hormone disorder.

The results of this example demonstrate that OCN15 can be applied to ovary-related reproductive diseases caused by hyperandrogen, including menstrual cycle disorder, polycystic ovary syndrome, androgenic phenotype, and abnormal metabolism caused by hormone disorder such as abnormal glucose metabolism, obesity, and osteoporosis.

In addition, the hair condition of the mice and the androgen receptor expression in the epidermal layer of the mice were observed on day 60 of modeling. The results are shown in FIG. 6, and it can be seen from the comparison of the 3 groups of mice that hyperandrogen leads to hair loss on the neck and back skin of the mice, and OCN15 can significantly improve the phenomenon (FIG. 6A). This is probably due to the fact that hyperandrogen leads to a significant increase of androgen receptor AR expression in the epidermal layer of the neck and back skin of the female mice, which in turn leads to hair loss, while OCN15 can significantly reduce DHT-caused significant increase of androgen receptor AR expression in the epidermal layer of the neck and back skin of the female mice (FIG. 6B), which is also consistent with hair loss condition of the neck and back of the mice.

The results of this example demonstrate that OCN15 can be applied to symptoms associated with hyperandrogen, such as female alopecia.

### Example 7: Effect of Ovary-Targeting Polypeptide on Oocyte Post-Ovulation Aging

Wild type female mice were intraperitoneally injected with 5 IU PMSG (pregnant mare serum gonadotropin), followed by an intraperitoneal injection of 5 IU hCG (human chorionic gonadotropin) 48 h later for superovulation. Oocytes were collected after 14 h, and cumulus cells were removed using hyaluronidase. Then, the oocytes were randomly divided into the Aging group and the Aging + OCN15 group and cultured in M16 medium for aging. The Control group was aged for 0 h, the Aging group for 12 h, and the Aging + OCN15 group in M16 containing the polypeptide (1 µg/mL) for 12 h.

The experimental results are shown in FIG. 7, and by comparing the three groups, OCN15 can ameliorate the fragmentation and degeneration caused by *in vitro* aging of oocytes (FIG. 7A). OCN15 can ameliorate the zona pellucida sclerosis caused by *in vitro* aging of oocytes (FIG. 7B). OCN15 can improve the low fertilization rate caused by *in vitro* aging of oocytes, with the rate approaching that with 0 h of aging, showing no significant difference.

The results of this example demonstrate that OCN15 can be applied to protect the oocyte quality, delay the aging process, and improve the low fertilization rate caused by aging. It can be applied to the field of assisted reproduction, and improves the preservation time and the preservation quality of oocytes and fertilized ova. Meanwhile, it is expected that OCN15 has an effect of ameliorating the *in vitro* aging of oocytes, and thus, it can be used for diseases induced by abnormalities caused by oocyte aging, such as abnormal oocyte number, decreased oocyte quality, and ovum aging.

In conclusion, the mediating polypeptide provided herein can be used for increasing the fertilization window time of oocytes and treating symptoms caused by hormone disorder such as ovary-related reproductive diseases, abnormal metabolism, alopecia, etc.

### Example 8: Effect of Ovary-Targeting Polypeptide Knockout on Serum Hormone Level in Mouse Superovulation Process

Wild type female mice and OCN15 KO mice were intraperitoneally injected with 5 IU PMSG, followed by an intraperitoneal injection of 5 IU hCG 48 h later for superovulation. Mice were sacrificed prior to injection, at PMSG24h, at PMSG48h, and at hCG12h, respectively, and serum was taken to detect the sex hormone level.

The experimental results are shown in FIG. 8, and it can be seen from the comparison with the WT mice that there was no change in estrogen levels during superovulation (left panel of FIG. 8), while at PMSG48h, the progesterone level significantly increased in the OCN15 KO mice compared with the WT mice (right panel of FIG. 8). At the PMSG48h time point, most follicles were stimulated to develop to the dominant follicle stage awaiting ovulation, and the progesterone at this stage was mainly secreted by the granulosa cells of the large follicles. This indicates that more follicles in the OCN15 KO mice responded to PMSG stimulation and developed to the stage ready for ovulation compared with the WT mice. Therefore, OCN15 knockout may lead to the overactivation of mouse follicles, potentially causing premature ovarian failure. This can prove that the ovary-targeting polypeptide provided herein can be used for treating premature ovarian failure.

### Example 9: Effect of Ovary-Targeting Polypeptide on Hyperandrogen-Induced Mouse Liver Lipid Droplet Accumulation

The method for the induction of the mouse PCOS model by hyperandrogen DHT is described in Example 6. After modeling, the mouse liver was taken, fixed overnight by 4% paraformaldehyde (PFA), dehydrated by alcohol gradient, embedded by paraffin, sectioned (5 µm), and subjected to HE staining for morphological observation.

The experimental results are shown in FIG. 9, and it can be seen from the comparison with the liver morphology of the WT mice that the liver cells of the DHT model mice became larger, indicating that more lipid droplets were accumulated in the cytoplasm, which was alleviated in the DHT + OCN15 treated group.

It is thereby demonstrated that the ovary-targeting polypeptide of the present disclosure can alleviate the hyperandrogen-induced mouse liver lipid droplet accumulation and is used for treating fatty liver.

The above descriptions are only specific embodiments of the present invention, not all of the embodiments. Any equivalent modifications of the technical solutions of the present invention, which are made by those skilled in the art by reading the present specification, are covered by the claims of the present invention.

## Claims

1. An ovary-targeting polypeptide, wherein the polypeptide sequence comprises a sequence represented by formula M1-Za-M2 or Za, wherein:
M1 and M2 are each independently a single or multiple amino acid polypeptide segment or are absent; and
Za is Tyr-Leu-X1-X2-X3-X4-Gly-Ala-X5-X6-Pro-X7-Pro-Asp-X8-Leu-Glu-Pro-Thr, wherein
X1 is Asn or Tyr or Asp or is absent,
X2 is Asn or Gln or His or Pro or Ser or is absent,
X3 is Gly or Trp or is absent,
X4 is Leu or is absent,
X5 is Pro or Ser,
X6 is Ala or Val,
X7 is Tyr or Ser or Ala, and
X8 is Pro or Thr,
preferably, wherein Za is selected from one of:
| | |
|---|---|
| YLGASVPSPDPLEPT | SEQ ID NO: 1; |
| YLNNGLGAPAPYPDPLEPT | SEQ ID NO: 2; |
| YLYQWLGAPVPYPDPLEPT | SEQ ID NO: 3; |
| YLYQWLGAPVPYPDTLEPT | SEQ ID NO: 4 |
| YLYQWLGAPAPYPDPLEPT | SEQ ID NO: 5 |
| YLDHWLGAPAPYPDPLEPT | SEQ ID NO: 6 |
| YLDPGLGAPAPYPDPLEPT | SEQ ID NO: 7 |
| YLDHGLGAPAPYPDPLEPT | SEQ ID NO: 8 |
| YLDQGLGAPAPAPDPLEPT | SEQ ID NO: 9 |
| and YLDSGLGAPVPYPDPLEPT | SEQ ID NO: 10. |

2. A derivative of an ovary-targeting polypeptide, wherein the derivative is a product obtained by performing a conventional modification on an amino acid side chain group, an amino terminal, and a carboxyl terminal of the polypeptide according to claim 1, or a product obtained by attaching a tag for peptide or protein detection or purification to the polypeptide according to claim 1, or a product obtained by performing isotope labeling modification; and the conventional modification is fluorophore modification, phosphorylation modification, disulfide bond cyclization modification, biotin labeling modification, photosensitizer modification, azide modification, PEG modification, methylation modification, fluorescence quencher modification, protein conjugation modification, small molecule compound modification, amination modification, amidation modification, hydroxylation modification, carboxylation modification, carbonylation modification, alkylation modification, acetylation modification, esterification modification, or glycosylation modification, wherein
preferably, the tag is His₆, GST, EGFP, MBP, Nus, HA, IgG, FLAG, c-Myc, or ProfinityeXact;
preferably, the modification on the amino terminal and the carboxyl terminal is selected from an N-terminal acetylation modification and a C-terminal amination modification of the polypeptide;
preferably, the modification on the side chain is selected from a modification on R group of the amino acid side chain in the polypeptide;
preferably, a fluorescent dye used in the fluorophore modification is selected from AMCA, FITC, Rhodamine, Cy3, Cy5, Cy5.5, Cy7, AIE, and ICG, and the modification can be used for fluorescence detection;
preferably, the phosphorylation modification is selected from a combination of one or more of p-Ser, p-Thr, and p-Tyr;
preferably, the glycosylation modification is selected from a combination of one or more of Ser, Asn, Thr, and Tyr;
preferably, the nitration modification is selected from a combination of one or more of Tyr;
preferably, the biotin in the biotin labeling is selected from D-biotin, biotin hydrazide, photoreactive biotin, and biotin-dUTP; and
preferably, an isotope used in the isotope labeling is selected from a combination of one or more of 13C, 14C, 14N, 15N, 2H, 3H, 180, 32P, 32S, 34S, 35S, 36S, 35Cl, 37Cl, 1251, and 131I.

3. A polynucleotide encoding the polypeptide according to claim 1 or the derivative according to claim 2.

4. A vector comprising the polynucleotide according to claim 3.

5. A host cell transfected with the vector according to claim 4.

6. Use of the polypeptide according to claim 1 or the derivative according to claim 2 in preparing an ovary-targeting detection reagent, ovary-targeting drug, or ovary-targeting tool, wherein
preferably, the tool is selected from a carrier or a formulation, more preferably a carrier, a formulation, or a kit for achieving a targeting effect by conjugating the polypeptide or the derivative thereof described herein, more preferably the formulation is a liposome or a nanoparticle.

7. Use of the polypeptide according to claim 1 or the derivative according to claim 2 in preparing a drug or detection reagent capable of targeting ovaries, wherein
preferably, the drug and the detection reagent include detection products prepared using the polypeptide according to claim 1 and/or the derivative according to claim 2 as an antigen, and more preferably, the detection products are detection reagents, kits, or test strips.

8. An ovary-targeting carrier, wherein the carrier comprises the polypeptide according to claim 1 and/or the derivative according to claim 2 as a targeting agent, wherein
preferably, the carrier is selected from a liposome, a nanoparticle, an exosome, a biodegradable high molecular substance, and an antibody-ligand; and
preferably, the carrier contains a component for treating an ovary-related reproductive disease.

9. An ovary-targeting drug, wherein the drug is a conjugate of the polypeptide according to claim 1 and/or the derivative according to claim 2 with an active component.

10. An ovary-targeting polypeptide probe, wherein the polypeptide probe is prepared by conjugating the polypeptide according to claim 1 and/or the derivative according to claim 2 with a fluorescent dye through an organic chemical reaction, wherein
preferably, the fluorescent dye is selected from dyes having a fluorophore in the near-infrared region, and more preferably, the dyes having a fluorophore in the near-infrared region are selected from AIE, Cy5, Cy7, and ICG.

11. Use of the polypeptide according to claim 1 and/or the derivative according to claim 2 in preparing a drug or reagent for treating or ameliorating symptoms of an ovary-related reproductive disease, wherein the ovary-related reproductive disease is selected from an ovary-related reproductive disease caused by germ cell abnormality, an ovary-related reproductive disease caused by hormone disorder, and aging and related symptoms caused by hormone disorder;
or use in preparing a drug or reagent for preventing oocyte aging *in vivo* or *in vitro,* wherein
preferably, the hormone disorder is an above-normal level of androgen;
preferably, the ovary-related reproductive disease caused by the germ cell abnormality is an ovary-related reproductive disease caused by oocyte aging and/or zona pellucida sclerosis of oocytes, and more preferably, the ovary-related reproductive disease caused by the germ cell abnormality is selected from abnormal oocyte number, decreased oocyte quality, and ovum aging;
preferably, the ovary-related reproductive disease caused by the hormone disorder is selected from menstrual cycle disorder, polycystic ovary syndrome, androgenic phenotype, hair loss, abnormal metabolism, ovarian insufficiency, premature ovarian failure, and ovulation failure; and more preferably, the androgenic phenotype is selected from acne, hirsutism, and alopecia;
preferably, the abnormal metabolism caused by the hormone disorder is selected from abnormal glucose metabolism, abnormal lipid metabolism, and more preferably is hyperglycemia, insulin resistance, obesity, fatty liver, and osteoporosis; and
preferably, the aging and related symptoms caused by the hormone disorder include perimenopausal period and climacteric syndromes.

12. An assisted reproduction article,wherein the assisted reproduction article comprises the polypeptide according to claim 1 and/or the derivative according to claim 2, wherein
preferably, the assisted reproduction article is a protective solution for oocytes and/or fertilized ova in the assisted reproduction process.
